# EUROPEAN PATENT SPECIFICATION

(11) **EP 1 443 987 B1**
(45) Date of publication and mention of the grant of the patent: **23.02.2011**
(21) Application number: 02801769.7
(22) Date of filing: 15.10.2002
(51) Int. Cl.: A61M 1/16, A61M 1/34, A61M 1/36

(54) **Plasmapheresis filter device and apparatus for therapeutic apheresis**
Plasmapherese-Filtervorrichtung und Gerät für die therapeutische Apherese
Dispositif et appareil filtre de plasmaphérèse utilisés dans l'aphérèse thérapeutique

(30) Priority: 17.10.2001 US 981783; 13.08.2002 US 219082
(43) Date of publication of application: 11.08.2004
(73) Proprietor: Transvivo, Inc., Napa, CA 94558 (US)
(72) Inventor: GORSUCH, Reynolds, G., Yountville, CA 94599 (US); PETERS, Harold, W., Martinez, CA 94553 (US); HANDLEY, Harold, H, JR., Novato, CA 94949 (US)
(74) Representative: Popp, Eugen
(86) International application number: PCT/US2002/033173
(87) International publication number: WO 2003/033054

(56) References cited:
- US-A- 5 152 743
- US-A1- 2002 087 109

## Description

### Background of the Invention

In PCT publication WO 01/78805A1 entitled "Specialized Hollow Fiber Membranes for *In-Vivo* Plasmapheresis and Ultrafiltration," there are disclosed elongated hollow microporous fibers having an asymmetrical fiber wall characterized by having a lower mass density adjacent to the inner wall surface extending along the interior lumen of the fiber and a higher mass density adjacent to the outer wall surface. Such a fiber wall morphology and pore structure provide unique characteristics necessary for separating blood plasma and/or plasma water *in-vivo* where continuous extraction of cell-free plasma or ultrafiltered plasma water and its associated toxins is carried out within the blood vessel of a patient or animal. Conventional hollow fibers or filter membranes such as those used in dialysate filter devices are unable to successfully perform *in-vivo*, intravascular plasma separation, becoming clogged within a very short period of time, e.g., minutes, as proteinaceous materials, blood platelets, and other components rapidly occlude the membrane pores. Moreover, conventional dialysate hollow fiber membrane filters do not perform satisfactorily *in-vivo* because of the relatively high flow rate of blood at the exterior fiber surface and relatively low lumen pressure as compared to dialysate filter apparatus conditions in which plasma separation is carried out at relatively low flow rates and high trans-membrane pressures. For example, typical *in-vivo* blood flow within a vena cava is about 2.5 L per minute, while blood flow through typical dialysate filter apparatus is nearly stagnant, e.g., about 0.42 ml per minute per fiber. Intravascular trans-membrane pressure is typically about 50 mm Hg or less, as compared to 100-300 mm Hg used in extracorporeal dialysate filters. Conventional dialysate filter membranes have little structural strength which, although acceptable in an encapsulated dialysate filter environment external to the body, are not suitable for introvascular use.

In the field of medicine, the term "therapeutic apheresis" refers to techniques for treating diseases using the patient's blood. Current medical practice extracts whole blood from the patient and, as a first stage, separates the plasma from the blood *ex-vivo* by centrifugal or membrane separation, and in a second stage treats the separated plasma by various techniques. The treated plasma and blood are recombined *ex-vivo* and returned to the patient. In the simplest procedure the separated plasma including the pathogenic macromolecules is discarded and substitution fluids such as fresh frozen plasma and albumen solution are re-infused to the patient.

In all of the aforesaid and currently practiced therapeutic apheresis procedures, whole blood must be removed from the body and processed in two *ex*-*vivo* stages. However, removal and treatment of whole blood has major disadvantages. Whole blood removal results in the necessity to heparinize or anticoagulate the patient to minimize clotting in the *ex*-*vivo* circuit and apparatus. Such treatment is counter-indicated in most surgical patients and deleterious to others due to consequential damage to blood components and the removal of vital blood components unrelated to the therapy. Removing and treating whole blood *ex-vivo* dictates that the procedure be a "batch" or intermittent process with attendant loss of efficiency and confinement of the patient to a clinical setting where support systems and machinery are available. Removal of whole blood also exposes the patient to contamination by viral and/or bacterial infection from nosocomial sources, and removal of erythrocytes, platelets and other large cellular blood components exposes them to risk of damage due to mechanical and chemical exposure to non-biocompatible surfaces of *ex-vivo* apparatus.
EP 1 351 725 is considered as prior art with respect to only the novelty of the present application, and describes a filter device for implantation into a blood vessel for carrying out in-vivo plasma separation. This document describes a device in which a single elongated tube is used for connection with a plurality of micro-porous fibres. US 5,152,743 relates to an apparatus for separation of blood cholesterol. This device utilises a plurality of micro-porous fibres which are positioned within a vein of the patient, such that filtration can take place.

### Summary of the Invention

The present invention provides a filter device in accordance with independent claim 1. Further preferred embodiments are given in the dependent claims.

The claimed invention can be better understood in view of the embodiments of a filter device described hereinafter. In general, the described embodiments describe preferred embodiments of the invention. The attentive reader will note, however, that some aspects of the described embodiments may extend beyond the scope of the claims. To the respect that the described embodiments indeed extend beyond the scope of the claims, the described embodiments are to be considered supplementary background information and do not constitute definitions of the invention *per se.* This relates, in particular, to the option of a single elongated hollow tube device. This also holds for the subsequent "Brief Description of the Drawings" as well as the "Detailed Description of the Preferred Embodiments."

The present invention relates to a filter device for being implanted in a blood vessel for carrying out *in-vivo* plasma separation incorporating a plurality of elongated hollow fibers having an asymmetrical fiber wall morphology in which the inner wall surface along the interior fiber lumen has a lower mass density and the fiber wall adjacent to the outer wall surface has a higher mass density. The device comprises a plurality of elongated hollow conduits or tubes to which opposite ends of each of the fibers are secured so that the interior of the one ore more hollow tubes communicates with the interior of each of the elongated hollow fibers. In a preferred embodiment, the device comprises a pair of elongated hollow tubes joined along their length with a first end of each of the hollow fibers secured to and communicating with the interior of one of the hollow tubes, and the second end of each of the fibers secured to and communicating with the interior of the other hollow tube. A plasma or plasma water extraction catheter includes a multiple lumen catheter, preferably a triple lumen catheter, secured to a proximal end of the one or more hollow tubes and communicating with the tube interior for directing blood plasma or plasma water passing through the fiber wall and into the fiber lumen to extracorporeal treatment or collection apparatus or equipment.

### Brief Description of the Drawings

Fig. 1 is a top view of a preferred embodiment of the filter device having a pair of elongated substantially parallel hollow tubes joined together along their length, showing distal and proximal end segments;

Fig. 2 is an enlarged sectional view of the filter device of Fig. 1 along the lines A-A showing a single elongated hollow fiber secured to the hollow tubes;

Fig. 3 is an enlarged side view of a portion of a filter device of the type illustrated in Fig. 1 showing four elongated hollow fibers secured along the hollow tubes;

Figs. 4 and 5 are sectional and side views of another filter device embodiment;

Fig. 6 is a sectional view of a triple lumen catheter illustrating the catheter interior;

Fig. 7 is a scanning electron microscopy (SEM) image of a cross-section of a typical elongated hollow fiber used in the filter device at 100 µm magnification showing the asymmetrical wall structure between the inner and outer fiber wall surface;

Fig. 8 is a SEM cross-section of a fiber of Fig. 7 at a magnification of 400 µm.

Fig. 9 is a schematic illustration of a preferred embodiment of apparatus for carrying out therapeutic apheresis;

Fig. 10 schematically illustrates one embodiment of therapeutic apheresis apparatus using plasma exchange; and

Fig. 11 schematically illustrates a therapeutic apheresis apparatus embodiment using double, cascade filtration.

### Detailed Description of the Preferred Embodiments

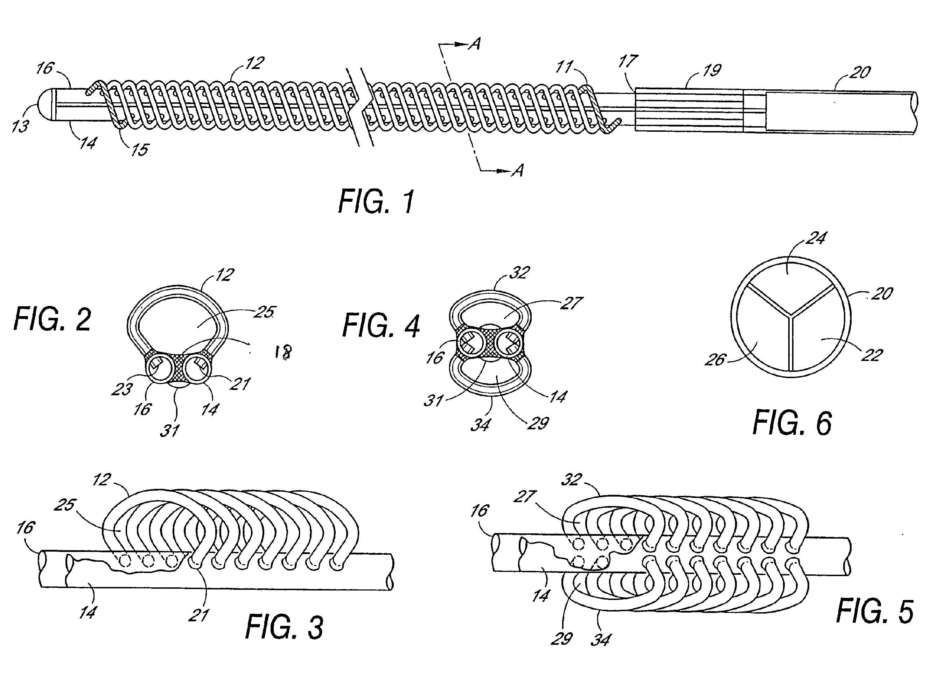

In the preferred embodiment illustrated in Figs. 1-3, a pair of elongated hollow tubes are joined side-by-side lengthwise to form the core of the filter device. The two elongated hollow core tubes 14 and 16 terminate at a distal end with a distal end plug or cap 13 formed of a material that seals the open tube ends. The tubes and end cap may be made of any suitable biocompatible material, for example, medical grade extruded urethane tubes. Other biocompatible materials include synthetic rubbers, polycarbonate, polyethylene, polypropylene, nylon, etc. The elongated hollow tubes may be secured together using suitable bonding material 18, adhesive compositions, etc., for example, a UV curable adhesive applied along the length between the two tubes. The length and diameter of the filter device may be selected to accommodate the vessel or vein in which it is to be implanted. Accordingly, the diameter and length of the one or more elongated hollow tubes forming the central core of the filter device are selected. A suitable tube length is between about 15 cm and about 25 cm, and preferably between about 18 cm and about 22 cm. Where a pair of core tubes is used as shown in the preferred embodiment, an outer diameter of each tube of between about 1 mm and about 3 mm is suitable. A detectable marker component 31, e.g., a radio opaque material may also be bonded to the device, for example, in bonding material 18 extending along the length of the tubes to assist in implanting and/or monitoring the device especially during insertion and removal.

The elongated hollow microporous fibers used in the filter device are the asymmetrical wall fibers disclosed in WO 01/78805. The morphology of the fiber walls is asymmetrical between the inner fiber lumen and the outer fiber wall which is in direct contact with the blood flowing in the vasculature in which the device is implanted. The filtration performance of such a device is a function of the filter surface of the exposed fibers whereby consideration is given to use larger diameter fibers and to maximize the number of fibers. Thus, it is desirable to use as many individual fibers along the hollow core tubes of the filter device as is practical while maintaining separation of the individual fibers to provide for fluid flow therebetween, and to maximize the amount of outer fiber surface exposed to blood flowing along the length of the filter device. Moreover, the fibers are secured along the length of the hollow tubes in such a manner as to form a fluid flow space between the fibers and the tubes. Again, however, the length of the filter device as well as the overall cross-sectional dimension are tailored or dictated by the blood vessel in which the device is to be used so as to avoid substantial interference with blood flow through the vessel while at the same time be efficient to achieve the intended flow rate of separated plasma.

In a preferred embodiment, the ends of each of the fibers are offset longitudinally relative to one another as illustrated in Figs. 1-3. As shown, elongated hollow fiber 12 has a first end 21 secured in first elongated hollow tube 14 and second end 23 secured in second hollow tube 16. In the specific device illustrated, the longitudinal spacing between the first and second ends of each fiber is a three-hole or three-fiber offset, e.g., about 0.5 cm. However, with intervals between the adjacent fiber ends of between about 0.1 cm and about 1.0 cm, offsets between first and second fiber ends may be between about 0.3 cm and about 3.0 cm, by way of example. With such offsets between first and second fiber ends, a straight line extending between the ends of a fiber forms an acute angle with an elongated axis of either or both of the elongated hollow tubes, and whereby the fibers also extend lengthwise between their ends along an angle other than 90° relative to the axes of the elongated hollow tubes. The acute angle preferably is between about 45° and about 85°. However, other fiber angles including 90° are not precluded and may be used where desired. In another preferred embodiment shown in Fig. 1, the proximal and distal fibers 11 and 15 located at each end of the filter device are filled with polyurethane or other biocompatible synthetic resin composition. These solid fibers at the ends of the row of fibers protect the adjacent hollow fibers from potential damage caused by mechanical stress during catheter insertion and removal.

In an example of assembly of a filter device, the elongated hollow core tubes 14 and 16 are joined as previously described and holes are drilled at the desired spacing along each of the two tubes. The holes may be drilled along opposite sides of the two tubes, and preferably are spaced at regular intervals of between about 0.1 cm and about 1.0 cm, and more preferably between 0.1 cm and about 0.3 cm. In a device as illustrated in Figs. 1-3, 6 fibers/cm are used and the interval or spacing between fiber ends along each of the tubes is approximately 1.66 mm. However, other practicable fiber spacing may be used, for example, between about 4 and about 8 fibers/cm and preferably between 5 and 7 fibers/cm of the length of the hollow tubes. The fibers may be secured in the spaced holes by any suitable method. For example, a first fiber end is inserted in a first hole in one of the tubes, the tubes are rotated 180°, and a second end of the fiber inserted in a first hole in the other tube. The procedure is repeated until all fiber ends are inserted in the holes along the two joined tubes. A wire or other elongated member may be inserted along the interior of each of the core tubes during assembly to provide a uniform limit or stop for the fiber ends along the respective hollow tube interior passageways. The fibers are bonded to the tubes and the joints between the fibers and the tubes sealed using a suitable adhesive or potting compound and the wires are removed. In the specific example of a filter device shown in Fig. 1, 118 active hollow fibers and 2 filled end fibers are spaced at 6 fibers/cm along 20.4 cm of the tubes. Each fiber is about 1.5 mm long.

Figs. 4 and 5 illustrate an alternative embodiment in which fibers are positioned on two sides of the filter device. Fibers 32 and 34 extend at opposite sides of the device whereby first and second ends of each of the fibers are secured along two rows along each of the tubes. As shown in Figs. 2-5, the fibers are arched to form a space between the fibers and the elongated tubes. In Figs. 2 and 3, a space 25 is formed by the arched fibers, and in Figs. 4 and 5, two spaces 27 and 29 are formed by the arched fibers on both sides of the filter device. The length of the fibers may be selected to accommodate the desired filter surface, as well as the desired cross-sectional dimension of the filter device as previously discussed. Suitable fiber lengths are between about 1 mm and about 4 mm to provide sufficient space between the arched fibers and the hollow tubes without distorting the fibers which could cause undesirable strains along the fiber walls or otherwise compromise fiber performance. The location of first and second fiber ends of the embodiment illustrated in Figs. 4 and 5 may be as described for the embodiment of Figs. 2 and 3.

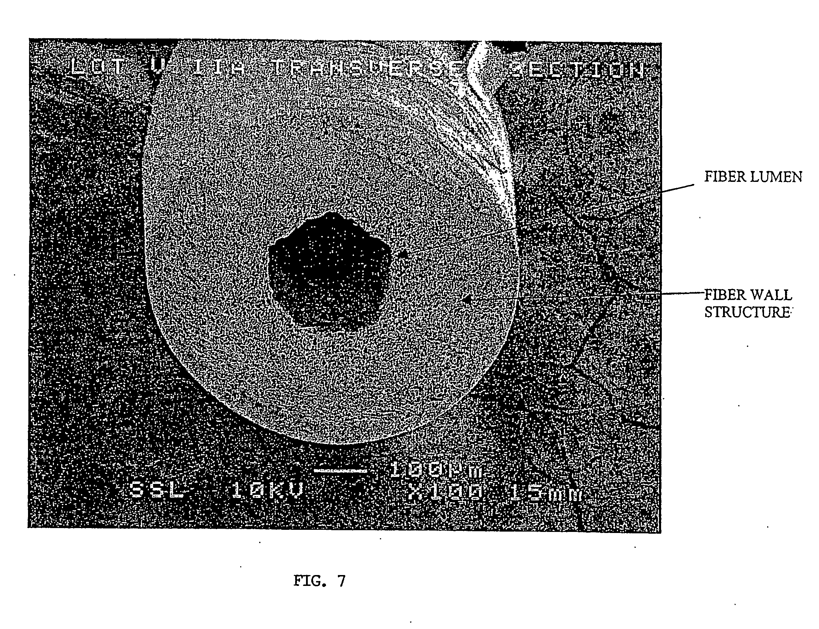

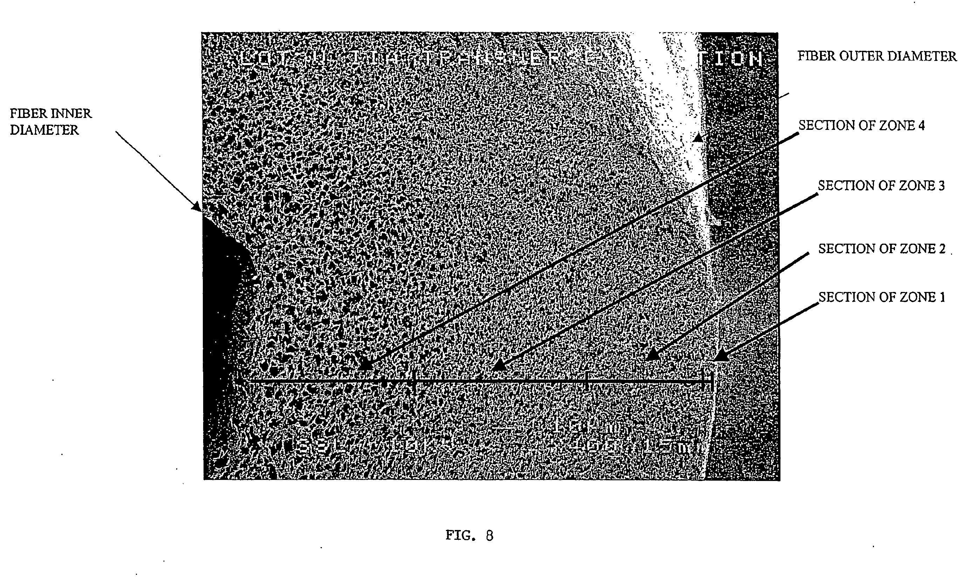

The fiber wall structure of the elongated microporous fibers is asymmetrical between the inner wall surface extending along the interior fiber lumen and the outer fiber wall surface exposed to blood in the vessel in which the filter device is implanted. The fiber wall at or adjacent to the outer wall surface has a higher mass density than the mass density adjacent to or at the inner wall surface. The mass density is a function of the average nominal pore size. Such asymmetric fiber wall morphology is illustrated in Figs. 7 and 8, Fig 7 showing a scanning electron microscopy (SEM) image of a cross-section of the fiber at 100 µm magnification. Fig. 8 shows a portion of the Fig. 7 fiber wall cross-section at a magnification of 400 µm. It will be observed that the structure of the fiber from the outer surface to the lumen is a continuous change in mass density whereby the pore size gradually changes between these fiber wall surfaces. However, it is convenient to describe the different mass density as sections or zones of the wall area having an average nominal pore size or average pore diameter, each zone having a different average nominal pore size. The walls may be characterized by two or more zones, for example 2, 3, or 4 or more mass density zones. The hollow fibers shown in Figs. 7 and 8 are also shown and described in the aforesaid publication WO 01/78805. In the fibers, the outer surface of the membrane, zone 1, has the highest mass density characterized by smaller average pore diameters. The outer zone forms the fiber interface with the permeate blood flow by determining filtration characteristics including the composition and components of separated plasma and controlling fiber membrane performance. Thus, zone 1 is the principle filtration portion of the fiber wall for controlling the trans-membrane flux (TMF) for excluding even the smallest cells in the blood, the platelets, having a diameter of about 1 µm. Nominal average pore diameters in zone 1 are between about 0.3 µm and about 1 µm, and preferably range from about 0.4 µm to about 0.8 µm. A preferred filtration sizing has a cutoff of about 0.6 µm to about 0.8 µm. Zones 2 and 3 are designed to decrease the flow path tortuosity and maintain the structural integrity required of the fiber exposed to physical conditions within the body. Pore size distribution in these zones ranges gradually from about 0.8 µm to about 1.2 µm and from about 1.2 µm to about 2.0 µm, respectively. Zone 2, having some flux-controlling pores, is principally to provide structural strength to the fiber as well as acting as a conduit for exudate flow to zone 3, also providing structure and enlarged pores for reducing the hydraulic resistance and providing a fluid conduit to the fiber lumen. The interior zones have little filtration function. Zone 4, representing the largest area having relatively large voids and pore diameters with little solid structure, has the primary function of a major reduction of hydraulic resistance through the membrane and defines the fiber inner lumen surface. Nominal average pore diameters in this lowest mass density zone are between about 1 µm and about 60 µm, and preferably between about 2 µm and about 6 µm. A typical fiber as shown has an OD of about 650 µm, an ID of about 250 µm and a wall thickness of about 250 µm. However, such dimensions are by way of example only. The fiber wall morphology, voids and pores may be further observed in WO 01/78805, with figures illustrating the structures at magnifications of 1,000 µm and 5,000 µm.

The elongated microporous fibers used in the filter device may be produced using biocompatible polymers including those produced from polyurethane, polypropylene, polysulfone (polyethersulfone), polycarbonate, nylon, polyimide, as well as other synthetic resins known to those skilled in the art. A preferred polymer is polysulfone, and more preferably a polyethersulfone/polyethylene oxide copolymer with a polyethylene glycol solvent or a polysulfone modified with polyethylene oxide-polyethylene glycol copolymer. Such polysulfone fibers are produced in the presence of polymer dopes, core fluids, and coagulation fluids using processes including membrane spinning methods which achieve the desired product. Examples of such additive materials used in the polymerization process, spinning process and/or fiber membrane production include polyvinyl pyrrolidone, N-methyl pyrrolidone, dimethyl acetomide, dimethyl sulfoxide, and mixtures of two or more such materials. Such polysulfone fibers have been found to have the least detrimental characteristics that influence protein membrane interaction such as crystallinity, ionic groups, hydrogen bonding groups and hydrophobic sites. Specific methods for producing the aforesaid polymers and fibers are known to those skilled in the art and disclosed, for example, in PCT Publication WO 90/04609.

The filter device is used for carrying out *in*-*vivo* plasmapheresis in combination with a multiple lumen catheter, preferably a triple lumen catheter as illustrated in Fig. 6. The catheter is of a suitable length to provide for implanting or installing the filter device into the appropriate vessel of the patient, e.g., the inferior vena cava, between the diaphragm and the iliac junction via the femoral vein, jugular vein or subclavian vein. The catheter 20 may be secured to the proximal end 17 of the filter device 10 by a suitable method, e.g., using a suitable adhesive and an injection-molded connector 19. The catheter 20 has an access lumen 26 which is in open fluid communication with the interior of elongated hollow tubes 14 and 16 of the filter device. Return lumen 22 is occluded or blocked off at the distal end of the catheter 20, and is provided with one or more ports through the catheter wall near the distal end of the catheter whereby treated plasma may be returned to the patient. Backflush lumen 24 is also in open fluid communication with the interior of the hollow tubes 14 and 16 through which periodic backflush fluid is directed for preventing occlusion of the hollow fiber membrane caused by blood components. Such backflush procedure and apparatus are discussed in detail in publication WO 02/053210 A2. The proximal end of the triple lumen catheter is secured to tubing components of a plasma separation system. The system includes plasma treatment apparatus for removing and/or separating selected plasma components and a fluid control assembly for directing plasma from the catheter to the treatment apparatus and return to the patient. The fluid control assembly also includes a pump for pumping plasma from the catheter to the treatment apparatus, a source of backflush fluid and a pump for pumping backflush fluid to the backflush lumen of the catheter. The fluid control apparatus also includes a microprocessor/controller for operating the pumps and controlling plasma flow rates and backflush fluid pressures, and backflush pumping intervals. The plasma treatment apparatus may be a single or multiple stage dialysate filter assembly or cascade membrane filters, absorbent cartridges, specialized adsorbent columns, chemical process or extraction assembly, or combinations, known to those skilled in the art.

Examples of medical applications for which the filter device described herein may be used include the following: therapeutic apherisis applications including plasma exchange, cascade protein separation and cascade protein removal or modification; fluid management application for congestive heart failure both acute and chronic; tissue engineering applications including online generation of media for bioreactor from xenogenic, allogenic, and autogenic sources; continuous renal replacement therapy (CRRT) for both acute and chronic kidney failure; edema prevention therapies for MODS (multiple organ dysfunction syndrome); cytokine removal or modification in therapy for septic shock or SIRS (systemic inflammatory response syndrome); plasma extraction from peritoneal ascites; intermittent hemodialysis (IHD) or hemodiafiltration; and ARDS (acute respiratory distress syndrome) therapy by reduction of pulmonary edema and physiological pulmonary dead space. Additional uses for the filter device of the present invention will be evident to those skilled in the art.

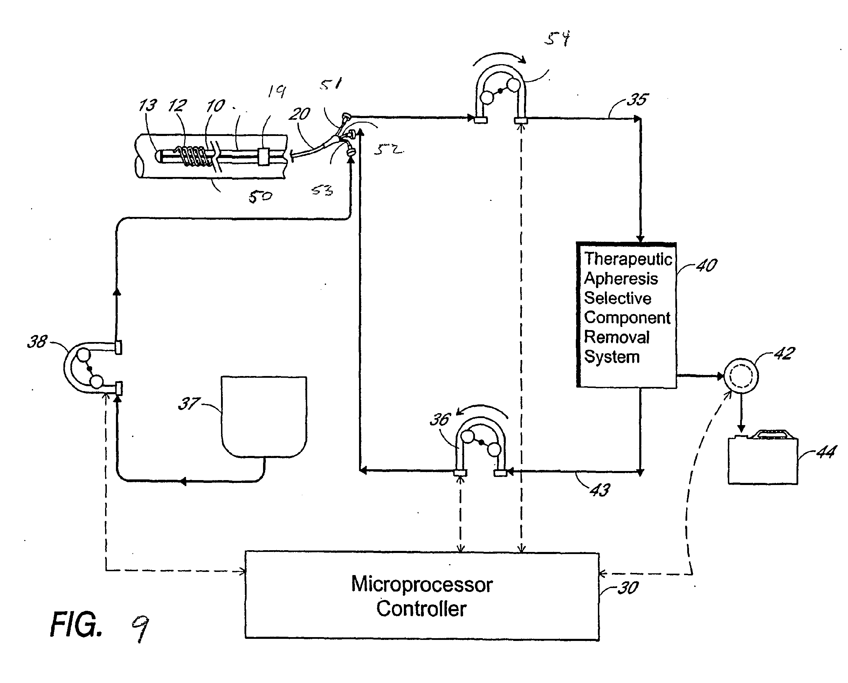

A preferred embodiment of an apparatus using the aforesaid filter device for carrying out therapeutic apheresis is schematically illustrated in Fig. 9. The apparatus includes a filter device 10, a triple lumen catheter 20, a therapeutic apheresis selective component removal apparatus 40, a fluid control assembly including tubing and pumps, and a microprocessor/controller 30. Veins suitable for implanting the filter include the superior or inferior vena cava or the subclavian vein. In the drawing, the filter device 10 is shown implanted in the inferior vena cava 50. A triple lumen catheter 20 is secured to the proximal end of the filter with connector 19. Triple lumen catheter 20 is in fluid communication with the interior of the filter device with the three catheter lumens connected to tubing for directing outgoing plasma, return plasma, and backflush fluid. Referring also to Figs. 1, 2 and 6, plasma separated from whole blood through the microporous fibers 12 of the filter device are directed through access lumen 26 and first tubing 51 to selective component apparatus 40. Plasma is separated from whole blood within the blood vessel in which the filter device is inserted using trans-membrane pressure (TMP) supplied by access pump or first pump 54, a positive displacement volumetric pump that operates to regulate pressure and control trans-membrane pressure and plasma volume removal rate.

Plasma from the filter device is pumped to the therapeutic apheresis selective component removal apparatus 40 for selectively removing disease-related components such as toxins, antibodies, proteins, pathogens including bacteria, virus, etc., and other disease-related substances desired to be removed. Plasma components and solutes removed from the treated plasma are directed to a container 44. An effluent pump 42 is optional and may be advantageously used for assisting in controlling the rate of disease components removed by providing controlled trans-membrane pressure across filter membranes of the selective component removal apparatus. Plasma is returned to the patient via tubing 43 at a rate controlled by pump 36. The tubing 43 is in fluid communication with plasma return tube 52 which is connected to plasma return lumen 22 of triple lumen catheter 20 (Fig. 5).

Examples of selective component removal apparatus used for therapeutic apheresis include plasma exchange components, centrifugal or membrane-separation filters, such as disclosed in U.S. Patent No. 5,605,627, cascade or multiple filtration membranes and columns, cartridges having components for absorbing (adsorbing) proteins and specific disease-related components, and activated charcoal cartridges. Other examples of useful selective component removal components include specialized columns utilizing materials such as cross-linked polyvinyl alcohol gel beads or microporous cellulose beads for removing specific amino acid ligands and antibodies. Further examples of selective component removal apparatus are chemical process systems for specialized uses such as heparin precipitation, plasma cyrofiltration, and salt-amino acid co-precipitation, and the like. Chemical process apparatus for effectively neutralizing disease related components in the plasma may also be used. These and other selective component removal apparatus and technologies are described in Therapeutic Apheresis, Official Journal of the International Society for Apheresis, Vol. 1-6, Blackwell Science Inc., "Present Status of Apheresis Technologies", e.g. Vol. 1, No. 2, May, 1997, pp. 135-146. Combinations of two or more of any of the aforesaid apparatus may also be used.

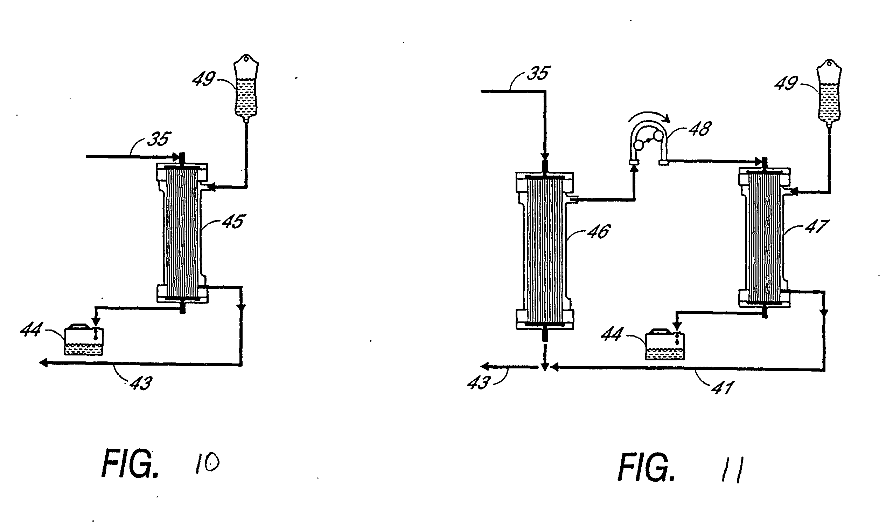

Fig. 10 illustrates a plasma exchange apparatus 45 for separating plasma components and for delivering fresh plasma from supply source 49. The plasma exchange rate may be selected as a function of the plasma removal rate by proportioning the rate of operation of access pump 34 to effluent pump 42, as shown in Fig. 9.

Fig. 11 schematically illustrates an example of selective component removal apparatus showing a cascade filter comprising a first stage filter 46 and a second stage filter 47. A pump 48 is used for directing fluid plasma from the first stage filter to the second stage filter. A source of make-up plasma liquid 49 may be used, if desired, for introducing substitution fluids such as fresh plasma which is combined with the treated plasma to be returned to the patient via tubing 41 and 43. Container 44 receives and collects discarded plasma fluid containing disease-related components, such as toxins, etc. as previously described. In a single stage treatment apparatus, the use of a make-up plasma liquid is also optional as is effluent pump 42 shown in Fig. 1 and cooperating with selective component removal apparatus 40 for directing fluid and components to be discarded. Again, following treatment in selective component removal apparatus 40, plasma is returned to the patient via piping 43 and positive displacement pump 36 to plasma return tube 52 which is in fluid communication with plasma return lumen 22 of triple lumen catheter 20.

An apparatus using cartridges or columns for absorbing or adsorbing disease-related components may also be used for treating separated plasma. Such apparatus may be configured like or similar to that illustrated in Figs. 10 and 11 in which the columns shown incorporate absorbing or adsorbing filters comprising materials capable of absorbing selected disease-related components such as discussed herein. Again, such an apparatus may include a source of fresh plasma to be directed to the patient, if desired.

A preferred apparatus shown in Fig. 9 includes backflush fluid reservoir 37, backflush pump 38 and backflush tube 53 communicating with a backflush lumen of the triple lumen catheter. Backflush pump 38 is selectively and periodically operated to provide backflush fluid flow for substantially cleansing the pores of the fiber membrane of the filter device. Such a backflush cycle is preferably operated at high trans-membrane pressure and low volume and at relatively short injection times for backflushing whereby the membrane pores are temporarily expanded and flushed to dislodge adhered proteins, thereby restoring pore integrity and density of the virtual filter area for improved performance after each backflush cycle.

Fluid control of plasma within the apparatus may be controlled using a microprocessor/controller operatively communicating with the positive displacement volumetric pumps for controlling trans-membrane pressure in the filter device and selective component removal apparatus, plasma removal rate, plasma return rate and backflush pressure and rate. Such fluid control and management may be selected, tailored or designed for slow, continuous acute fluid removal. For example, operation of the system may be used for controlling plasma extraction rate from blood to achieve removal of 1-2 L of plasma water over a 24-hour period. The fluid control assembly may also include volume sensors, pressure sensors, blood leak detectors and air detectors connected to the piping and reservoirs as desired. As illustrated in Fig. 9, the microprocessor/controller 30 is operatively connected to the pumps. Similarly, the microprocessor/controller operates for controlling backflush pump 38 and plasma is returned at a selected rate by controlling pump 36. The microprocessor/controller may be programmed for flow rates designed to a the prescribed patient therapy.

Examples of diseases and disorders for which therapeutic apheresis may be used and the pathogenic substances removed using the methods and apparatus of the invention include those described in Therapeutic Apherisis, Vol. 1, No. 2, 1997. The list is not intended to be exhaustive, and other diseases and substances may also be treated. Moreover, the methods and apparatus described herein may also be used in drug treatment, for example in drug overdose cases, where one or more toxic substances in the blood stream may be removed using the aforesaid methods and apparatus. These as well as others advantages will be evident to those skilled in the art.

## Claims

1. A filter device for being implanted in a blood vessel for carrying out *in-vivo* plasma separation comprising:
a plurality of elongated hollow tubes (14, 16) and a plurality of elongated microporous fibers (12) having an interior lumen extending along the length thereof, each fiber having a first and second end (21, 23) wherein each end is secured to an attachment point on a different one of said elongated hollow tubes, wherein the interior lumen of each of the fibers communicates with the interior of two of said hollow tubes, and wherein the fiber wall morphology of each of the elongated microporous fibers is asymmetrical between the inner wall surface extending along the interior fiber lumen and the outer wall surface, said fiber wall having a higher mass density zone adjacent to the outer wall surface and a lower mass density zone adjacent to the inner wall surface, said higher mass density zone having a smaller average nominal pore size than the average nominal pore size in the lower mass density zone, and wherein said fibers are capable of separating plasma from whole blood by passing plasma through the fiber wall from the outer wall surface to the inner wall surface.

2. A filter device of Claim 1, comprising one or more first elongated hollow tubes and one or more second elongated hollow tubes extending substantially parallel along the length thereof, and wherein a first end of each of said elongated microporous fibers is secured to a first hollow tube and a second end of each of said fibers is secured to a second hollow tube whereby the interior fiber lumen of each fiber communicates with the interior of a first and a second hollow tube.

3. A filter device of Claim 2, comprising two of said elongated hollow tubes, each of said tubes having a plurality of holes spaced apart along a substantial portion of the length thereof, each hole receiving a first or a second end of an elongated microporous fiber.

4. A filter device of Claim 2, wherein the first and second ends of said elongated microporous fibers are secured to said first and second elongated hollow tubes in generally straight rows along the side of each of said tubes.

5. A filter device of Claim 4, wherein the first hollow tube extends along a first axis and the second hollow tube extends along a second axis substantially parallel with said first axis, and wherein the first ends of said elongated microporous fibers are secured to said first hollow tube along a generally straight first row, and the second ends of said elongated microporous fibers are secured to said second hollow tube along a generally straight second row substantially parallel with said first row.

6. A filter device of Claim 5, wherein the distance between said first and second rows is greater than the distance between said first and second axes.

7. A filter device of Claim 6, wherein each of said fibers are generally bowed along its length between said first and second ends to form an arch spaced apart from said elongated hollow tubes and forming a passageway therebetween.

8. A filter device of Claim 7, wherein said elongated microporous fibers comprise first and second fibers, said first fibers forming a first arch of spaced fibers extending over a first portion of said device, said second fibers forming a second arch intending over a second portion of said device, opposite the first portion, said first and second arches spaced apart from said elongated hollow tubes to form passageways therebetween.

9. A filter device of Claim 8, wherein first ends of first elongated microporous fibers are secured along a first row on a first hollow tube and second ends of first fibers are secured along a first row on a second hollow tube, and first ends of second fibers are secured along a second row on the first hollow tube and second ends of second fibers are secured along a second row on the second hollow tube, whereby said first and second fibers form opposite first and second arches, respectively of spaced fibers along said device.

10. A filter device of Claim 5, 6, 7, 8 or 9, wherein the first and second ends of said elongated microporous fibers are secured to said first and second hollow tubes, respectively, at substantially regular intervals.

11. A filter device of Claim 10, wherein said regular intervals are between about 0.1 cm and about 1.0 cm.

12. A filter device of Claim 10, wherein said regular intervals are between about 0.1 cm and about 0.3 cm.

13. A filter device of Claim 5, 6, 7, 8 or 9, wherein the length of each of said elongated microporous fibers is between about 1 cm and about 4 cm.

14. A filter device of Claim 11, wherein lie length of each of said elongated microporous fibers is between about 1 cm and about 4 cm.

15. A filter device of Claim 5, 6, 7, 8 or 9, wherein the first end of each elongated microporous fiber is offset longitudinally from the second end of each said fiber along the length of said elongated hollow tubes whereby a straight line extending through the first and second end of a fiber forms an acute angle with one of said axes.

16. A filter device of Claim 15, wherein the space between adjacent fibers is between about 0.1 cm and about 1.0 cm.

17. A filter device of Claim 15, wherein said acute angle is between about 45° and about 85°.

18. A filter device of Claim 15, wherein the length of each hollow tube is between about 10 cm and about 25 cm.

19. A filter device of Claim 15, wherein the outer diameter of each hollow tube is between about 1 mm and about 3 mm.

20. A filter device of Claim 15, wherein the length of each hollow tube is between about 10 cm and about 25 cm, wherein the length of each elongated microporous fiber is between about 1 mm and about 4 mm, wherein the space between adjacent fibers is between about 0.1 cm and about 0.3 cm, and wherein said acute angle is between about 45° and about 85°.

21. A filter device of Claim 15, having between 4 and 8 fibers/cm of the length of said hollow tubes.

22. A filter device of Claim 1, wherein the fiber wall structure comprises a continuous change in mass density between the inner and outer surfaces of the fiber.

23. A filter device of Claim 22, wherein a lower mass density zone is **characterized by** a nominal average pore diameter of between about 1 µm and about 60 µm.

24. A filter device of Claim 22 or 23, wherein a higher mass density zone is **characterized by** a nominal average pore diameter of between about 0.3 µm and about 1 µm.

25. A filter device of Claim 22, wherein the nominal average pore diameter in a lower mass density zone is between about 2 µm and about 6 µm.

26. A filter device of Claim 22, wherein the nominal average pore diameter in a higher mass density zone is between about 0.4 µm and about 0.8 µm.

27. Apparatus for carrying out therapeutic apheresis comprising:
- an implantable filter device of Claim 1;
- a triple lumen catheter secured to the proximal end of the filter device having one or more lumens in-fluid communication with the interior of said hollow tubes and a plasma return lumen; and
- therapeutic apheresis apparatus for removing and/or separating selected disease-related components from the separated plasma and tubing for directing plasma between said catheter and the selective component removal apparatus,

28. Apparatus of Claim 27, wherein said triple lumen catheter comprises a first lumen and a second lumen in fluid communication with the interior of said hollow tubes and a third lumen comprising said plasma return lumen.

29. Apparatus of Claim 27, including:
- a fluid control assembly comprising first tubing in fluid communication with said first lumen of said catheter and a first fluid pump co-operating therewith for directing plasma from said filter device, second tubing in fluid communication with said second lumen of said catheter and a second pump cooperating therewith for directing backflush fluid into said filter device, and third tubing in fluid communication with said third lumen of said catheter for directing plasma from the therapeutic apheresis apparatus to a patient; and
- control apparatus operatively communicating with said first and second pumps for controlling the operation thereof, respectively.

30. Apparatus of Claim 29, including a third jump cooperating with said third tubing and in control connection with said control apparatus.

31. Apparatus of Claim 29, including a source of backflush fluid cooperating with said second tubing.

32. Apparatus of Claim 29, wherein said controller comprises a microprocessorcontroller including software programmed for operating said apparatus.

33. Apparatus of Claim 27 or 29, wherein said therapeutic apheresis apparatus comprises plasma exchange, multiple stage filtration, one or more absorption columns or cartridges or one or more reactors containing compositions for reacting with and neutralizing disease-related plasma components.

34. Apparatus of Claim 32, including apparatus for substituting fresh plasma for a portion of separated plasma.

## Patentansprüche

1. Filtervorrichtung zur Implantierung in ein Blutgefäß zur Durchführung von einer In-vivo-Plasmatrennung, welche enthält:
eine Mehrzahl von langgestreckten hohlen Röhren (14, 16) und eine Mehrzahl von langgestreckten mikroporösen Fasern (12), welche ein Innenlumen haben, welches sich entlang der Länge davon erstreckt, wobei jede Faser ein erstes und ein zweites Ende (21, 23) hat, wobei jedes Ende an einem Befestigungspunkt an einer unterschiedlichen von den langgestreckten hohlen Röhren befestigt ist, wobei das Innenlumen von jeder von den Fasern mit dem Inneren von zwei von den hohlen Röhren in Verbindung steht, und wobei die Form von der Faserwand von jeder von den langgestreckten mikroporösen Fasern zwischen der Innenwandfläche, welche sich entlang des inneren Faserlumens und der Außenwandfläche erstreckt, asymmetrisch ist, wobei die Faserwand angrenzend zu der Außenwandfläche eine höhere Massendichtenzone und angrenzend zu der Innenwandfläche eine geringere Massendichtenzone hat, wobei die höhere Massendichtenzone eine kleinere mittlere nominale Porengröße als die mittlere nominale Porengröße in der geringeren Massendichtenzone hat, und wobei die Fasern dazu fähig sind, Plasma vom gesamten Blut zu trennen, indem Plasma durch die Faserwand von der Außenwandfläche zur Innenwandfläche überführt wird.

2. Filtervorrichtung nach Anspruch 1, welche eine oder mehrere erste langgestreckte hohle Röhren enthält, und wobei sich eine oder mehrere zweite langgestreckte hohle Röhren im Wesentlichen parallel entlang der Länge davon erstrecken, und wobei ein erstes Ende von jeder von den langgestreckten mikroporösen Fasern an einer ersten hohlen Röhre befestigt ist, und wobei ein zweites Ende von jeder von den Fasern an einer zweiten hohlen Röhre befestigt ist, wobei das innere Faserlumen von jeder Faser mit dem Inneren von einer ersten und einer zweiten hohlen Röhre in Verbindung steht.

3. Filtervorrichtung nach Anspruch 2, welche zwei von den langgestreckten hohlen Röhren enthält, wobei jede von den Röhren eine Mehrzahl von Löchern hat, welche entlang -eines wesentlichen Abschnittes von der Länge davon beabstandet sind, wobei jedes Loch ein erstes oder ein zweites Ende von einer langgestreckten mikroporösen Faser aufnimmt.

4. Filtervorrichtung nach Anspruch 2, bei welcher das erste und zweite Ende von den langgestreckten mikroporösen Fasern entlang der Seite von jeder von den Röhren bei im Allgemeinen geraden Zeilen an der ersten und zweiten langgestreckten hohlen Röhre befestigt ist.

5. Filtervorrichtung nach Anspruch 4, bei welcher sich die erste hohle Röhre entlang einer ersten Achse erstreckt und sich die zweite hohle Röhre entlang einer zweiten Achse erstreckt, welche im Wesentlichen parallel zur ersten Achse ist, und wobei die ersten Enden von den langgestreckten mikroporösen Fasern entlang von einer im Allgemeinen geraden ersten Zeile an der ersten hohlen Röhre befestigt sind, und wobei die zweiten Enden von den langgestreckten mikroporösen Fasern entlang von einer im Allgemeinen geraden zweiten Zeile mit der zweiten hohlen Röhre befestigt sind, welche im Wesentlichen parallel zur ersten Zeile ist.

6. Filtervorrichtung nach Anspruch 5, bei welcher der Abstand zwischen der ersten und zweiten Zeile größer ist als der Abstand zwischen der ersten und zweiten Achse.

7. Filtervorrichtung nach Anspruch 6, bei welcher jede von den Fasern entlang von ihrer Länge zwischen dem ersten und zweiten Ende im Allgemeinen bogenförmig ist, um einen Bogen auszubilden, welcher von den langgestreckten hohlen Röhren beabstandet ist und dazwischen einen Durchgang ausbildet.

8. Filtervorrichtung nach Anspruch 7, bei welcher die langgestreckten mikroporösen Fasern eine erste und eine zweite Faser enthalten, wobei die ersten Fasern einen ersten Bogen von beabstandeten Fasern ausbilden, welche sich über einen ersten Abschnitt von der Vorrichtung erstrecken, wobei die zweiten Fasern einen zweiten Bogen ausbilden, welcher sich über einen zweiten Abschnitt von der Vorrichtung, gegenüberliegend zum ersten Abschnitt, erstreckt, wobei der erste und zweite Bogen von den langgestreckten hohlen Röhren beabstandet sind, um Durchgänge dazwischen auszubilden.

9. Filtervorrichtung nach Anspruch 8, bei welcher die ersten Enden von den ersten langgestreckten mikroporösen Fasern entlang von einer ersten Zeile an einer ersten hohlen Röhre befestigt sind, und wobei die zweiten Enden von ersten Fasern entlang von einer ersten Zeile an einer zweiten hohlen Röhre befestigt sind, und wobei die ersten Enden von den zweiten Fasern entlang von einer zweiten Zeile an der ersten hohlen Röhre befestigt sind, und wobei zweite Enden von zweiten Fasern entlang von einer zweiten Zeile an der zweiten hohlen Röhre befestigt sind, wobei die ersten und zweiten Fasern erste und zweite gegenüberliegende Bögen, jeweils von beabstandeten Fasern entlang von der Vorrichtung, ausbilden.

10. Filtervorrichtung nach Anspruch 5, 6, 7, 8 oder 9, bei welcher die ersten und zweiten Enden von den langgestreckten mikroporösen Fasern jeweils an der ersten und zweiten hohlen Röhre bei im Wesentlichen gleichen Abständen befestigt sind.

11. Filtervorrichtung nach Anspruch 10, bei welcher die gleichen Abstände zwischen ungefähr 0,1 cm und ungefähr 1,0 cm sind.

12. Filtervorrichtung nach Anspruch 10, bei welcher die gleichen Abstände zwischen ungefähr 0,1 cm und ungefähr 0,3 cm sind.

13. Filtervorrichtung nach Anspruch 5, 6, 7, 8 oder 9, bei welcher die Länge von jeder von den langgestreckten mikroporösen Fasern zwischen ungefähr 1 cm und ungefähr 4 cm ist.

14. Filtervorrichtung nach Anspruch 11, bei welcher die Länge von jeder von den langgestreckten mikroporösen Fasern zwischen ungefähr 1 cm und ungefähr 4 cm ist.

15. Filtervorrichtung nach Anspruch 5, 6, 7, 8 oder 9, bei welcher das erste Ende von jeder langgestreckten mikroporösen Faser von dem zweiten Ende von jeder von den Fasern entlang der Länge von den langgestreckten hohlen Röhren in Längsrichtung versetzt ist, wobei eine gerade Linie, welche sich durch das erste und zweite Ende von einer Faser erstreckt, mit einer von den Achsen einen spitzen Winkel ausbildet.

16. Filtervorrichtung nach Anspruch 15, bei welcher der Abstand zwischen angrenzenden Fasern zwischen ungefähr 0,1 cm und ungefähr 1,0 cm ist.

17. Filtervorrichtung nach Anspruch 15, bei welcher der spitze Winkel zwischen ungefähr 45° und ungefähr 85° ist.

18. Filtervorrichtung nach Anspruch 15, bei welcher die Länge von jeder hohlen Röhre zwischen ungefähr 10 cm und ungefähr 25 cm ist.

19. Filtervorrichtung nach Anspruch 15, bei welcher der Außendurchmesser von jeder hohlen Röhre zwischen ungefähr 1 mm und ungefähr 3 mm ist.

20. Filtervorrichtung nach Anspruch 15, bei welcher die Länge von jeder hohlen Röhre zwischen ungefähr 10 cm und ungefähr 25 cm ist, wobei die Länge von jeder langgestreckten mikroporösen Faser zwischen ungefähr 1 mm und ungefähr 4 mm ist, wobei der Abstand zwischen angrenzenden Fasern zwischen ungefähr 0,1 cm und ungefähr 0,3 cm ist, und wobei der spitze Winkel zwischen ungefähr 45° und ungefähr 85° ist.

21. Filtervorrichtung nach Anspruch 15, welche zwischen 4 und 8 Fasern/cm der Länge von den hohlen Röhren hat.

22. Filtervorrichtung nach Anspruch 1, bei welcher der Faserwandaufbau zwischen der Innen- und Außenfläche von der Faser eine kontinuierliche Änderung in der Massendichte enthält.

23. Filtervorrichtung nach Anspruch 22, bei welcher eine geringere Massendichtenzone durch einen nominalen mittleren Porendurchmesser zwischen ungefähr 1 µm und ungefähr 60 µm **gekennzeichnet** ist.

24. Filtervorrichtung nach Anspruch 22 oder 23, bei welcher eine höhere Massendichtenzone durch einen nominalen mittleren Porendurchmesser zwischen ungefähr 0,3 µm und ungefähr 1 µm **gekennzeichnet** ist.

25. Filtervorrichtung nach Anspruch 22, bei welcher der nominale mittlere Porendurchmesser in einer geringeren Massendichtenzone zwischen ungefähr 2 µm und ungefähr 6 µm ist.

26. Filtervorrichtung nach Anspruch 22, bei welcher der nominale mittlere Porendurchmesser in einer höheren Massendichtenzone zwischen ungefähr 0,4 µm und ungefähr 0,8 µm ist.

27. Einrichtung zur Durchführung einer therapeutischen Apherese, welche enthält:
eine implantierbare Filtervorrichtung nach Anspruch 1;
einen Dreifach-Lumen-Katheder, welcher am proximalen Ende von der Filtervorrichtung befestigt ist, welcher einen oder mehrere Lumen hat, welche mit dem Inneren von den hohlen Röhren und einem Plasma-Rückgabe-Lumen in Fluidverbindung stehen; und
eine therapeutische Apherese-Einrichtung zur Entnahme und/oder zur Trennung ausgewählter krankheitsbezogener Anteile aus dem getrennten Plasma und zur Weiterleitung zum Leiten von Plasma zwischen dem Katheder und der Auswahl-Anteil-Entnahme-Einrichtung.

28. Einrichtung nach Anspruch 27, bei welcher der Dreifach-Lumen-Katheder ein erstes Lumen und ein zweites Lumen, welche mit dem Inneren von den hohlen Röhren in Fluidverbindung stehen, und ein drittes Lumen enthält, welches das Plasma-Rückgabe-Lumen enthält.

29. Einrichtung nach Anspruch 27, welche enthält:
eine Fluidsteueranordnung, welche eine erste Röhre, welche mit dem ersten Lumen von dem Katheder und einer Fluidpumpe, welche damit zusammenwirkt, um Plasma von der ersten Filtervorrichtung zu leiten, in Fluidverbindung steht, eine zweite Röhre, welche mit dem zweiten Lumen von dem Katheder und einer zweiten Pumpe, welche damit zusammenwirkt, um ein Zurückfließen des Fluids in die Filtervorrichtung zu leiten, in Fluidverbindung steht, und eine dritte Röhre, welche mit dem dritten Lumen von dem Katheder in Fluidverbindung steht, um Plasma von der therapeutischen Apherese-Einrichtung an einen Patienten zu leiten, enthält; und
eine Steuereinrichtung, welche betriebsbedingt jeweils mit der ersten und zweiten Pumpe zum Steuern des Betriebes davon in operativer Verbindung steht.

30. Einrichtung nach Anspruch 29, welche einen dritten Anstieg enthält, welcher mit der dritten Röhre zusammenwirkt, und mit der Steuereinrichtung in Steuerverbindung steht.

31. Einrichtung nach Anspruch 29, welche eine Quelle eines zurückfließenden Fluides enthält, welche mit der zweiten Röhre zusammenwirkt.

32. Einrichtung nach Anspruch 29, bei welcher die Steuerung eine Mikroprozessor-Steuerung enthält, welche eine Software enthält, welche zum Betreiben von der Einrichtung programmiert ist.

33. Einrichtung nach Anspruch 27 oder 29, bei welcher die therapeutische Apherese-Einrichtung einen Plasma-Austausch, eine mehrstufige Filterung, eine oder mehrere Absorptions-Spalten oder Behälter oder einen oder mehrere Reaktoren enthält, welche Zusammensetzungen zum Reagieren mit krankheitsbedingten Plasma-Anteilen und Neutralisieren derer enthält.

34. Einrichtung nach Anspruch 32, welche eine Einrichtung zum Ersetzen von frischem Plasma für einen Anteil von getrenntem Plasma enthält.

## Revendications

1. Dispositif de filtre destiné à être implanté dans un vaisseau sanguin pour réaliser une séparation de plasma *in vivo* comprenant :
une pluralité de tubes creux allongés (14, 16) et une pluralité de fibres microporeuses allongées (12) ayant une lumière intérieure s'étendant suivant leur longueur, chaque fibre comportant une première et une seconde extrémité (21, 23) où chaque extrémité est fixée à un point d'attache d'un tube différent parmi lesdits tubes creux allongés, où la lumière intérieure de chacune des fibres communique avec l'intérieur de deux desdits tubes creux, et où la morphologie de paroi de fibre de chacune des fibres microporeuses allongées est asymétrique entre la surface de paroi interne s'étendant le long de la lumière de fibre intérieure et la surface de paroi externe, ladite paroi de fibre ayant une zone de densité de masse supérieure adjacente à la surface de paroi externe et une zone de densité de masse inférieure adjacente à la surface de paroi interne, ladite zone de densité de masse supérieure ayant une plus petite taille de pore nominale moyenne que la taille de pore minimale moyenne dans la zone de densité de masse inférieure, et où lesdites fibres sont capables de séparer le plasma du sang entier en faisant passer le plasma à travers la paroi de fibre de la surface de paroi externe à la surface de paroi interne.

2. Dispositif de filtre selon la revendication 1, comprenant un ou plusieurs premiers tubes creux allongés et un ou plusieurs seconds tubes creux allongés s'étendant de façon sensiblement parallèle suivant leur longueur, et où une première extrémité de chacune desdites fibres microporeuses allongées est fixée à un premier tube creux et une seconde extrémité de chacune desdites fibres est fixée à un second tube creux, moyennant quoi la lumière de fibre intérieure de chaque fibre communique avec l'intérieur d'un premier et d'un second tube creux.

3. Dispositif de filtre selon la revendication 2, comprenant deux desdits tubes creux allongés, chacun desdits tubes comportant une pluralité de trous espacés le long d'une partie substantielle de leur longueur, chaque trou recevant une première ou une seconde extrémité d'une fibre microporeuse allongée.

4. Dispositif de filtre selon la revendication 2, dans lequel les première et seconde extrémités desdites fibres microporeuses allongées sont fixées auxdits premier et second tubes creux allongés en lignes généralement droites le long du côté de chacun desdits tubes.

5. Dispositif de filtre selon la revendication 4, dans lequel le premier tube creux s'étend le long d'un premier axe et le second tube creux s'étend le long d'un second axe sensiblement parallèle audit premier axe, et où les premières extrémités desdites fibres microporeuses allongées sont fixées audit premier tube creux le long d'une première ligne généralement droite, et les secondes extrémités desdites fibres microporeuses allongées sont fixées audit second tube creux le long d'une seconde ligne généralement droite sensiblement parallèle à ladite première ligne.

6. Dispositif de filtre selon la revendication 5, dans lequel la distance entre lesdites première et seconde lignes est supérieure à la distance entre lesdits premier et second axes.

7. Dispositif de filtre selon la revendication 6, dans lequel chacune desdites fibres est généralement courbée suivant sa longueur entre lesdites première et seconde extrémités pour former un arc espacé desdits tubes creux allongés et formant un passage entre elles.

8. Dispositif de filtre selon la revendication 7, dans lequel lesdites fibres microporeuses allongées comprennent des premières et secondes fibres, lesdites premières fibres formant un premier arc de fibres espacées s'étendant sur une première partie dudit dispositif, lesdites secondes fibres formant un second arc s'étendant sur une seconde partie dudit dispositif, à l'opposé de la première partie, lesdits premier et second arcs étant espacés desdits tubes creux allongés pour former des passages entre eux.

9. Dispositif de filtre selon la revendication dans lequel les premières extrémités des premières fibres microporeuses allongées sont fixées le long d'une première ligne sur un premier tube creux et les secondes extrémités des premières fibres sont fixées le long d'une première ligne sur un second tube creux; et les premières extrémités des secondes fibres sont fixées le long d'une seconde ligne du premier tube creux et les secondes extrémités des secondes fibres sont fixées le long d'une seconde ligne du second tube creux, moyennant quoi lesdites premières et secondes fibres forment des premier et second arcs opposés, respectivement de fibres espacées le long dudit dispositif.

10. Dispositif de filtre selon la revendication 5, 6, 7, 8 ou 9, dans lequel les premières et secondes extrémités desdites fibres microporeuses allongées sont fixées auxdits premier et second tubes creux, respectivement, à des intervalles sensiblement réguliers.

11. Dispositif de filtre selon la revendication 10, dans lequel lesdits intervalles réguliers sont compris entre environ 0,1 cm et environ 1,0 cm.

12. Dispositif de filtre selon la revendication 10, dans lequel lesdits intervalles réguliers sont compris entre environ 0,1 cm et environ 0,3 cm.

13. Dispositif de filtre selon la revendication 5, 6, 7, 8 ou 9, dans lequel la longueur de chacune desdites fibres microporeuses allongées est comprise entre environ 1 cm et environ 4 cm.

14. Dispositif de filtre selon la revendication 11, dans lequel la longueur de chacune desdites fibres microporeuses allongées est comprise entre environ 1 cm et environ 4 cm.

15. Dispositif de filtre selon la revendication 5, 6, 7, 8 ou 9, dans lequel la première extrémité de chaque fibre microporeuse allongée est décalée longitudinalement le long de la seconde extrémité de chacune desdites fibres suivant la longueur desdits tubes creux allongés, moyennant quoi une ligne droite s'étendant à travers la première et la seconde extrémité d'une fibre forme un angle aigu avec l'un desdits axes.

16. Dispositif de filtre selon la revendication 15, dans lequel l'espace entre les fibres adjacentes est compris entre environ 0,1 cm et environ 1,0 cm.

17. Dispositif de filtre selon la revendication 15, dans lequel ledit angle aigu est compris entre environ 45° et environ 85°.

18. Dispositif de filtre selon la revendication 15, dans lequel la longueur de chaque tube creux est comprise entre environ 10 cm et environ 25 cm.

19. Dispositif de filtre selon la revendication 15, dans lequel le diamètre externe de chaque tube creux est compris entre environ 1 mm et environ 3 mm.

20. Dispositif de filtre selon la revendication 15, dans lequel la longueur de chaque tube creux est comprise entre environ 10 cm et environ 25 cm, où la longueur de chaque fibre microporeuse allongée est comprise entre environ 1 mm et environ 4 mm, où l'espace entre les fibres adjacentes est compris entre environ 0,1 cm et environ 0,3 cm, et où ledit angle aigu est compris entre environ 45°et environ 85°.

21. Dispositif de filtre selon la revendication 15, comportant entre 4 et 8 fibres/cm de la longueur desdits tubes creux.

22. Dispositif de filtre selon la revendication 1, dans lequel la structure de paroi de fibre comprend un changement continu de densité de masse entre les surfaces interne et externe de la fibre.

23. Dispositif de filtre selon la revendication 22, dans lequel une zone de densité de masse inférieure est **caractérisée par** un diamètre de pore moyen nominal compris entre environ 1 µm et environ 60 µm.

24. Dispositif de filtre selon la revendication 22 ou 23, dans lequel une zone de densité de masse supérieure est **caractérisée par** un diamètre de pore moyen nominal compris entre environ 0,3 µm et environ 1 µm.

25. Dispositif de filtre selon la revendication 22, dans lequel le diamètre de pore nominal moyen dans une zone de densité de masse inférieure est compris entre environ 2 µm et environ 6 µm.

26. Dispositif de filtre selon la revendication 22, dans lequel le diamètre de pore nominal moyen dans une zone de densité de masse supérieure est compris entre environ 0,4 µm et environ 0,8 µm.

27. Appareil pour réaliser une aphérèse thérapeutique comprenant :
- un dispositif de filtre implantable selon la revendication 1 ;
- un cathéter à lumière triple fixé à l'extrémité proximale du dispositif de filtre comportant une ou plusieurs lumières en communication fluidique avec l'intérieur desdits tubes creux et une lumière de retour de plasma ; et
- un appareil d'aphérèse thérapeutique pour enlever et/ou séparer des composants sélectionnés liés à une maladie du plasma séparé et un tubage pour diriger le plasma entre ledit cathéter et l'appareil d'enlèvement de composant sélectif.

28. Appareil selon la revendication 27, dans lequel ledit cathéter à lumière triple comprend une première lumière et une deuxième lumière en communication fluidique avec l'intérieur desdits tubes creux et une troisième lumière comprenant ladite lumière de retour de plasma.

29. Appareil selon la revendication 27, comprenant :
- un ensemble de commande de fluide comprenant un premier tubage en communication fluidique avec ladite première lumière dudit cathéter et une première pompe de fluide coopérant avec celle-ci pour diriger un plasma depuis ledit dispositif de filtre, un deuxième tubage en communication fluidique avec ladite deuxième lumière dudit cathéter et une deuxième pompe coopérant avec celui-ci pour diriger un fluide de rétrobalayage dans ledit dispositif de filtre, et un troisième tubage en communication fluidique avec ladite troisième lumière dudit cathéter pour diriger un plasma de l'appareil d'aphérèse thérapeutique à un patient ; et
- un appareil de commande communiquant en fonctionnement avec lesdites première et deuxième pompes pour en commander le fonctionnement, respectivement.

30. Appareil selon la revendication 29, comprenant une troisième pompe coopérant avec ledit troisième tubage et en connexion de commande avec ledit appareil de commande.

31. Appareil selon la revendication 29, comprenant une source de fluide de rétrobalayage coopérant avec ledit deuxième tubage.

32. Appareil selon la revendication 29, dans lequel ladite unité de commande comprend un microprocesseur-unité de commande comprenant un logiciel programmé pour actionner ledit appareil.

33. Appareil selon la revendication 27 ou 29, dans lequel ledit appareil d'aphérèse thérapeutique comprend un échange de plasma, une filtration d'étage multiple, une ou plusieurs colonnes d'absorption ou des cartouches ou un ou plusieurs réacteurs contenant des compositions pour réagir avec et neutraliser des composants de plasma liés à une maladie.

34. Appareil selon la revendication 32, comprenant un appareil pour remplacer une partie du plasma séparé par du plasma frais.
